Europäisches Patentamt

European Patent Office

Office européen des brevets

(19)

(11) Veröffentlichungsnummer: **0 227 584 B1**

(12) **EUROPÄISCHE PATENTSCHRIFT**

(45) Veröffentlichungstag der Patentschrift: **29.05.91**

(51) Int. Cl.5: **C12M 1/00, C12P 5/02**

(21) Anmeldenummer: **86730174.9**

(22) Anmeldetag: **23.10.86**

(54) **Verfahren und Vorrichtung zur Trocknung von Wasserpflanzen.**

(30) Priorität: **27.12.85 DE 3546303**

(43) Veröffentlichungstag der Anmeldung:
**01.07.87 Patentblatt 87/27**

(45) Bekanntmachung des Hinweises auf die
Patenterteilung:
**29.05.91 Patentblatt 91/22**

(84) Benannte Vertragsstaaten:
**AT BE CH FR GB IT LI LU NL SE**

(56) Entgegenhaltungen:
**WO-A-81/03029**
**DE-A- 3 015 239**

**BIOTECHNOLOGY & BIOENGINEERING, Band
27, Nr. 6, Juni 1985, Seiten 905-908, John
Wiley & Sons, Inc., New York, US; S. VAIDY-
ANATHAN et al.: "Biogas production in
batch and semicontinuous digesters using
water hyacinth"**

**PATENT ABSTRACTS OF JAPAN, Band 7, Nr.
175 (C-179)[1320], 3. August 1983; & JP-A-58
81 790 (MITSUBISHI JUKOGYO K.K.)
17-05-1983**

(73) Patentinhaber: **MANNESMANN Aktiengesellschaft**
**Mannesmannufer 2**
**W-4000 Düsseldorf 1(DE)**

(72) Erfinder: **Bracker, Gerd-Peter, Dr.-Ing.**
**Weidengrund 18**
**W-4630 Bochum(DE)**
Erfinder: **Schnorr, Karl-Ernst**
**Niederau 6**
**W-6335 Lahnau 3(DE)**

(74) Vertreter: **Meissner, Peter E., Dipl.-Ing. et al**
**Patentanwaltsbüro Meissner & Meissner,**
**Herbertstrasse 22**
**W-1000 Berlin 33(DE)**

## Beschreibung

Die Erfindung bezieht sich auf ein Verfahren und eine Vorrichtung zur Trocknung von Wasserpflanzen wie Algen, Seetang, Wasserhyazinthen usw.

Durch die Eutrophierung ist es in vielen Gewässern zu einem starken Wachstum von Wasserpflanzen gekommen, die im Zuge der Gewässerreinigung entfernt werden müssen. Diese Wasserpflanzen haben außen eine schleimige Bakterienmasse und im Inneren neben der biologisch leicht fermentierbaren Zellflüssigkeit ein Gerüst aus Lignin und Zellulose, welches prinzipiell zur Herstellung von Dünge-, Heiz- oder Futtermitteln nutzbar ist. Die frisch geborgene Masse hat jedoch einen Wassergehalt von bis zu 95 Gewichts-%. Sie muß daher vor ihrer Weiterverwendung auf einen lufttrockenen Zustand (ca. 10 - 20 Gewichts-% Wasser) gebracht werden. Der hierzu bisher notwendige Aufwand war in vielen Fällen so hoch, daß eine wirtschaftliche Aufbereitung derartiger Wasserpflanzen nicht möglich war.

Der Erfindung liegt die Aufgabe zugrunde, ein Verfahren anzugeben, mit dem die Trocknung derartiger Pflanzenmassen unter möglichst weitgehendem Verzicht auf den Einsatz von Fremdenergie durchführbar ist.

Gelöst wird diese Aufgabe erfindungsgemäß durch ein Verfahren mit den Merkmalen des Patentanspruchs 1. Vorteilhafte Weiterbildungen des Verfahrens sind in den Unteransprüchen 2 - 12 angegeben. Eine Vorrichtung zur Durchführung des Verfahrens ist im Anspruch 13 gekennzeichnet. Die Ansprüche 14 - 20 beziehen sich auf vorteilhafte Weiterbildungen dieser Vorrichtung. Im folgenden wird die Erfindung näher erläutert.

Nach dem Bergen der Wasserpflanzen werden diese zunächst zerkleinert und homogenisiert und - zweckmäßigerweise in erwärmtem Zustand - einer Faulanlage zur anaeroben Fermentation der schleimigen Bakterienmasse zugeführt. In vielen Fällen kann die Faulung einstufig in einem Reaktor durchgeführt werden. Besonders bei größeren Anlagen ist es jedoch zweckmäßig, eine Trennung der Hydrolyse und der sauren Gärung von der Methanfaulung vorzunehmen, um den jeweiligen Bakterien optimale Umweltbedingungen zu geben. Das bei der anaeroben Fermentation entstehende Biogas kann zur vorherigen Erwärmung der in den Reaktor einzubringenden Wasserpflanzenmasse genutzt werden.

Nach diesem Fermentationsschritt wird der entstandene Schlamm vorentwässert, indem er z.B. mit Hilfe eines mechanischen Entwässerungsaggregates auf eine Restfeuchte von 50 - 75 Gewichts-% gebracht wird. Dabei entsteht eine feste Masse, die danach mit bereits getrocknetem Endprodukt so gemischt wird, daß der Wassergehalt dieser Mischung bei 30 - 40 Gewichts-%, vorzugsweise bei 35 Gewichts-% liegt. Durch diesen Prozeßschritt wird eine Masse gewonnen, die eine krümelige Struktur mit hohem Porenvolumen aufweist. Sie wird anschließend zum Austreiben des Zellwassers in Rotteanlagen oder auf Paletten in Würfeln so gelagert, daß die Luft möglichst unten ein-und oben austreten kann. Bei der Palettenlagerung von Würfeln kann der seitliche Luftzu- und -austritt z.B. durch eine rundumlaufende Schrumpffolie oder Wände aus Stahl- bzw. Alumniumblech verhindert werden. Während dieser Lagerung beginnt eine aerobe Fermentation, die einen Teil des Wassergehaltes der Mischung aufzehrt. Zusätzlich unterstützt die entstehende Wärme das Trocknen der Masse, ohne daß Fremenergie aufgewendet werden muß. Die in der Abluft noch enthaltene Wärme kann zur Erwärmung des Eingangsmaterials für die anaerobe Fermentation weitergenutzt werden.

Da das Kohlenstoff/ Stickstoff-Verhältnis der Mischung relativ hoch ist, kann zur Verbesserung der mikrobiellen Fermentation Stickstoff z.B. in Form von Harnstoff oder Kunstdünger zugemischt werden. Vorteilhaft ist es auch, die Belüftung der einzelnen Würfel durch Anordnung von Ventilatoren zu intensivieren. Hierdurch wird die Fermentations- und damit auch die Trocknungszeit herabgesetzt und gleichzeitig die Bildung anaerober Zonen unterbunden. Günstig ist es weiterhin, die Paletten mit einer Stapelvorrichtung so zu versehen, daß eine Übereinanderstapelung erfolgen kann. Hierdurch kann die Trocknung platzsparend und die Zwangsbelüftung effektiver durchgeführt werden. Ein weiterer Vorteil des Einsatzes von Paletten besteht darin, daß die getrocknete Masse direkt, d.h. ohne Zwischenbehandlung zum Verwender transportiert werden kann.

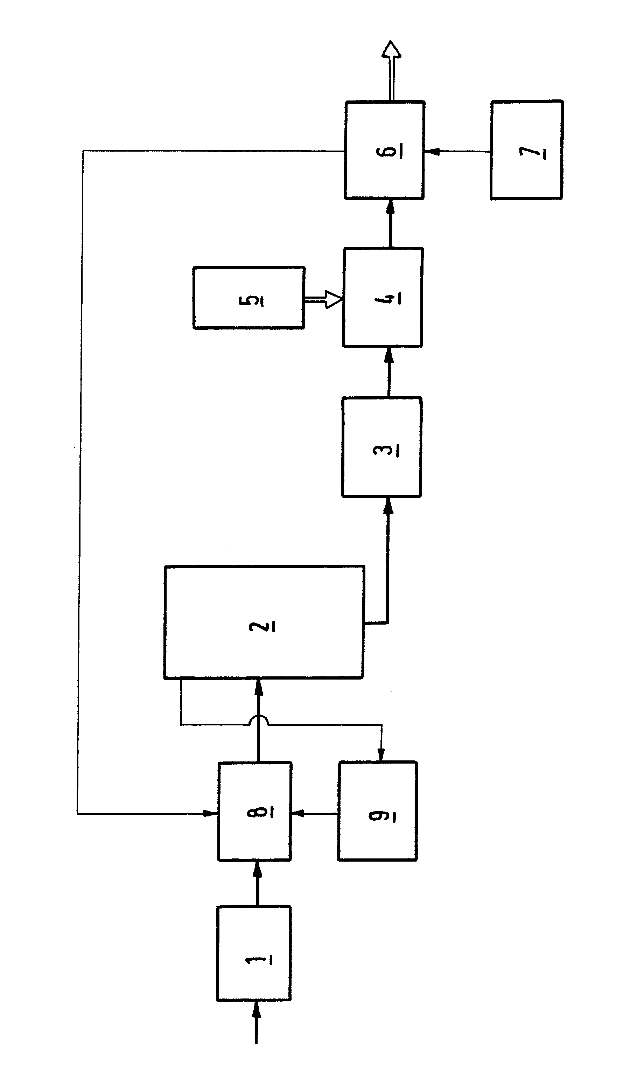

In der Figur ist der Verfahrensablauf anhand eines Anlagenschemas beispielhaft dargestellt. Die geborgene nasse Wasserpflanzenmasse gelangt zunächst in eine Zerkleinerungsvorrichtung 1. Nach Zerkleinerung und Homogenisierung tritt die Wasserpflanzenmasse in einen Wärmetauscher 8 ein und wird dort auf eine Temperatur erwärmt, die für die nachfolgende anaerobe Fermentation in dem Reaktor 2 vorteilhaft ist. Das in dem Reaktor 2 anfallende Biogas wird in eine Feuerungseinrichtung 9 gegeben, die zur Beheizung des Wärmetauschers 8 dient. Nach Durchlaufen des Reaktors 2 wird die Wasserpflanzenmasse in Form von Schlamm zunächst in die Entwässerungseinrichtung 3 (z.B. eine Zentrifuge) gegenen und gelangt danach in den Mischer 4. Dieser Mischer 4 ist mit einer Zuführeinrichtung 5 (z.B. Siloanlage) für die Zumischung von bereits getrocknetem Endprodukt verbunden. Das aus dem Mischer 4 herauskommende Material wird danach in eine Vorrichtung 6 zur aeroben Fermentation und Trocknung gegeben. Diese Vorrichtung 6 kann beispielsweise als automatisiertes geschlossenes Rottesilo ausgebildet sein; es ist aber ebenso möglich, aus der gemischten Pflanzenmasse Ballen zu erzeugen, die z.B. auf Paletten gelagert werden und ebenfalls eine aerobe Fermentation ermöglichen.

Durch eine Zwangsbelüftung von unten nach oben, die durch eine entsprechende Einrichtung 7 (z.B. Ventilator) unterstützt wird, ist eine aerobe Fermentation gewährleistet. Die dabei entstehende Wärme führt zur Verdunstung eines erheblichen Teiles des Zellwassergehaltes. Da die aus Vorrichtung 6 entweichende Abluft erwärmt ist, kann diese aufgefangen und in den Wärmetauscher 8 geleitet werden, um zu einer Erwärmung der frischen Wasserpflanzenmasse beizutragen.

**Ansprüche**

1. Verfahren zur Trocknung von zerkleinerten Wasserpflanzen unter Anwendung der aeroben Fermentation,
   **dadurch gekennzeichnet,**
   daß die Zerkleinerung der Wasserpflanzen in nassem Zustand erfolgt und die Wasserpflanzen anschließend einer anaeroben Fermentation unterworfen werden, daß der dabei entstehende Schlamm mechanisch auf eine Restfreuchte von 50 - 75 Gewichts-% entwässert wird und danach mit bereits getrocknetem Endprodukt zur Verbesserung des Porenvolumens in solchen Mengen gemischt wird, daß der Wassergehalt 30 - 40 Gewichts-% beträgt und daß diese Mischung anschließend der aeroben Fermentation unterworfen wird, wobei die Mischung mindestens so lange von Umgebungsluft durchströmt wird, bis die biologische Wasserumsetzung und die durch die hierbei entstehende Wärme geförderte Wasserverdunstung zu einer Trocknung auf 10 - 20 Gewichts-% Wasser geführt hat.

2. Verfahren nach Anspruch 1,
   **dadurch gekennzeichnet,**
   daß die anaerobe Fermentation in der Weise zweistufig erfolgt, daß die Hydrolyse und die saure Gärung getrennt von der Methanfaulung abläuft.

3. Verfahren nach Anspruch 1 oder 2,
   **dadurch gekennzeichnet,**
   daß dem Mischgut Stickstoff zugegeben wird.

4. Verfahren nach Anspruch 3,
   **dadurch gekennzeichnet,**
   daß der Stickstoff in Form von Harnstoff zugegeben wird.

5. Verfahren nach Anspruch 3,
   **dadurch gekennzeichnet,**
   daß der Stickstoff in Form von Kunstdünger zugegeben wird.

6. Verfahren nach einem der Ansprüche 1 - 5,
   **dadurch gekennzeichnet,**
   daß die Mischung zwangsbelüftet wird.

7. Verfahren nach einem der Ansprüche 1 - 6,
   **dadurch gekennzeichnet,**
   daß die Mischung auf Paletten in Würfelform gelagert wird, wobei die Würfelaußenflächen durch geschlossene Seitenwände begrenzt werden.

8. Verfahren nach Anspruch 7,
   **dadurch gekennzeichnet,**
   daß die auf Paletten ruhenden Mischungswürfel mittels einer Stapelvorrichtung übereinander gestapelt werden.

9. Verfahren nach einem der Ansprüche 7 oder 8,
   **dadurch gekennzeichnet,**
   daß die Würfel einzeln oder zusammen an eine Belüftungsanlage angeschlossen werden.

10. Verfahren nach einem der Ansprüche 1 - 9,
    **dadurch gekennzeichnet,**
    daß die zerkleinerten Wasserpflanzen vor Eintritt in die anaerobe Fermentationsstufe erwärmt werden.

11. Verfahren nach Anspruch 10,
    **dadurch gekennzeichnet,**
    daß die Abluft aus der Trocknungsstufe zur Erwärmung der zerkleinerten Wasserpflanzen benutzt wird.

12. Verfahren nach Anspruch 10 oder 11,
    **dadurch gekennzeichnet,**
    daß die Heizenergie des bei anaeroben Fermentation entstehenden Biogases zur Erwärmung der zerkleinerten Wasserpflanzen benutzt wird.

13. Vorrichtung zur Durchführung des Verfahrens nach Anspruch 1,
    **dadurch gekennzeichnet,**
    daß eine Zerkleinerungsvorrichtung (1) für Wasserpflanzen mit der Eingangsseite eines Reaktors (2) für eine anaerobe Fermentation verbunden ist, dessen Ausgangsseite an eine Entwässerungsvorrichtung (3) angeschlossen ist, daß hinter der Entwässerungsvorrichtung (3) eine Mischvorrichtung (4) vorgesehen ist, die eine Zuführeinrichtung (5) für getrocknete Wasserpflanzen aufweist, und daß die Ausgangsseite der Mischvorrichtung (4) mit einer Vorrichtung (6) zur aeroben Fermentation und Trocknung verbunden ist.

**14.** Vorrichtung nach Anspruch 13,
**dadurch gekennzeichnet,**
daß die Vorrichtung (6) als Ballenrotte ausgebildet ist.

**15.** Vorrichtung nach Anspruch 13,
**dadurch gekennzeichnet,**
daß die Vorrichtung (6) als geschlossenes automatisiertes Rottesilo ausgebildet ist.

**16.** Vorrichtung nach einem der Ansprüche 13 - 15,
**dadurch gekennzeichnet,**
daß die Vorrichtung (6) an eine Zwangsbelüftungseinrichtung (7) angeschlossen ist.

**17.** Vorrichtung nach einem der Ansprüche 13 - 16,
**dadurch gekennzeichnet,**
daß zwischen der Zerkleinerungsvorrichtung (1) und dem Reaktor (2) ein Wärmeaustauscher (8) zur Erwärmung der Wasserpflanzen angeordnet ist.

**18.** Vorrichtung nach Anspruch 17,
**dadurch gekennzeichnet,**
daß der Wärmeaustauscher (8) mit der Abluftseite der Vorrichtung (6) verbunden ist.

**19.** Vorrichtung nach Anspruch 17 oder 18,
**dadurch gekennzeichnet,**
daß die Biogasseite des Reaktors (2) mit einer Feuerungsanlage (9) zur Beheizung des Wärmeaustauschers (8) verbunden ist.

**20.** Vorrichtung nach einem der Ansprüche 13 - 19,
**dadurch gekennzeichnet,**
daß der Reaktor (2) als zweistufiger Faulreaktor ausgebildet ist.

**Claims**

**1.** Method for drying comminuted aquatic plants using
aerobic fermentation,
characterised in that the comminution of the aquatic plants takes place in the wet state and the aquatic plants are then subjected to anaerobic fermentation, that the resulting sludge is dewatered mechanically to a residual moisture content of 50 to 75% by weight, and is then mixed with already-dried end product to improve the pore volume, in such quantities that the water content is 30 to 40% by weight, and that this mixture is then subjected to aerobic fermentation, with surrounding air flowing through the mixture at least until the biological water reaction and the water evaporation promoted by the heat produced thereby has led to drying to 10 - 20% by weight water.

**2.** Method according to Claim 1,
characterised in that
the anaerobic fermentation takes place in two stages in that the hydrolysis and the acidic fermentation take place separately from the methane rotting.

**3.** Method according to Claim 1 or 2,
characterised in that
nitrogen is added to the mixed material.

**4.** Method according to Claim 3,
characterised in that
the nitrogen is added in the form of urea.

**5.** Method according to Claim 3,
characterised in that
the nitrogen is added in the form of fertiliser.

**6.** Method according to one of Claims 1 - 5,
characterised in that
the mixture is subjected to forced aeration.

**7.** Method according to one of Claims 1 - 6,
characterised in that the mixture is stored on pallets in cube form, with the outer cube surfaces being delimited by closed side walls.

**8.** Method according to Claim 7,
characterised in that
the mixture cubes resting on pallets are stacked one above the other by means of a stacking device.

**9.** Method according to one of Claims 7 or 8,
characterised in that
the cubes are connected, individually or together, to an aeration plant.

**10.** Method according to one of claims 1 - 9,
characterised in that
the comminuted aquatic plants are heated before entering the anaerobic fermentation stage.

**11.** Method according to claim 10,

characterised in that
the outgoing air from the drying stage is used to heat the comminuted aquatic plants.

12. Method according to Claim 10 or 11,
characterised in that
the heating energy of the biogas produced upon the anaerobic fermentation is used for heating the comminuted aquatic plants.

13. Apparatus for performing the method according to Claim 1,
characterised in that
a comminution device (1) for aquatic plants is connected to the entry side of a reactor (2) for anaerobic fermentation, the exit side of which is connected to a dewatering apparatus (3), that after the dewatering apparatus (3) there is provided a mixing apparatus (4) which has a supply means (5) for dried aquatic plants, and that the exit side of the mixing apparatus (4) is connected to an apparatus (6) for aerobic fermentation and drying.

14. Apparatus according to claim 13,
characterised in that
the apparatus (6) is designed as a bale gang.

15. Apparatus according to Claim 13,
characterised in that
the apparatus (6) is designed as a closed automated gang silo.

16. Apparatus according to one of Claims 13 - 15,
characterised in that
the apparatus (6) is connected to a forced aeration apparatus (7).

17. Apparatus according to one of Claims 13 - 16,
characterised in that
a heat exchanger (8) for heating the aquatic plants is located between the comminution apparatus (1) and the reactor (2).

18. Apparatus according to Claim 17,
characterised in that
the heat exchanger (8) is connected to the outgoing airside of the apparatus (6).

19. Apparatus according to Claim 17 or 18,
characterised in that
the biogas side of the reactor (2) is connected to a furnace unit (9) for heating the heat exchanger (8).

20. Apparatus according to one of Claims 13 - 19,
characterised in that
the reactor (6) is designed as a two-stage rotting reactor.

**Revendications**

1. Procédé de séchage de plantes aquatiques hachées, avec mise en oeuvre de la fermentation aérobie, caractérisé en ce que le hachage des plantes aquatiques se fait à l'etat humide et en ce que les plantes aquatiques sont ensuite soumises à une fermentation anaérobie, en ce que la boue générée lors de ce processus est déshydratée mécaniquement pour atteindre une humidité résiduelle de 50 - 75 % en poids et est ensuite mélangée à un produit final déjà séché en quantités telles que la teneur en eau se situe de 30 à 40 % en poids pour améliorer le volume poreux, et en ce que ce mélange est ensuite soumis à la fermentation aérobie, ledit mélange étant envahi par l'air ambiant au moins aussi longtemps que la conversion biologique de l'eau et l'évaporation de l'eau stimulée par la chaleur engendrée à cette occasion conduisent à un séchage réduisant la quantité d'eau à 10 - 20 % en poids.

2. Procédé selon la revendication 1, caractérisé en ce que la fermentation anaérobie s'opère en deux étapes de façon à séparer l'hydrolyse et la fermentation acide de la digestion méthanique.

3. Procédé selon la revendication 1 ou 2, caractérisé en ce que de l'azote est ajouté aux matières à mélanger.

4. Procédé selon la revendication 3, caractérisé en ce que l'azote est fourni sous forme d'urée.

5. Procédé selon la revendication 3, caractérisé en ce que l'azote est fourni sous forme d'engrais chimique.

6. Procédé selon l'une des revendications 1 - 5, caractérisé en ce que le mélange est soumis à une ventilation forcée.

7. Procédé selon l'une des revendications 1 - 6, caractérisé en ce que le mélange est stocké sous forme de cubes sur des palettes, les surfaces extérieures des cubes étant limitées par des parois latérales fermées.

8. Procédé selon la revendication 7, caractérisé en ce que les cubes de mélange reposant sur les palettes sont empilés les uns sur les autres à l'aide d'un dispositif d'empilage.

9. Procédé selon la revendication 8, caractérisé en ce que les cubes sont raccordés individuellement ou conjointement à une installation de ventilation.

10. Procédé selon l'une des revendications 1 - 9, caractérisé en ce que les plantes aquatiques hachées sont chauffées avant d'être engagées dans l'étape de fermentation anaérobie.

11. Procédé selon la revendication 10, caractérisé en ce que l'air dégagé provenant de l'étape de séchage est utilisé pour chauffer les plantes aquatiques hachées.

12. Procédé selon la revendication 10 ou 11, caractérisé en ce que l'énergie chauffante du biogaz généré lors de la fermentation anaérobie est utilisée pour chauffer les plantes aquatiques hachées.

13. Dispositif pour l'exécution du procédé selon la revendication 1, caractérisé en ce qu'un dispositif hacheur (1) pour les plantes aquatiques est raccordé au côté entrée d'un réacteur (2) pour la fermentation anaérobie, dont le côté sortie est raccordé à un dispositif de déshydratation (3), en ce qu'un dispositif mélangeur (4) présentant un dispositif d'alimentation (5) pour des plantes aquatiques séchées a été prévu derrière le dispositif de déshydratation (3), et en ce que le côté sortie du dispositif mélangeur (4) est raccordé à un dispositif (6) de fermentation aérobie et de séchage.

14. Dispositif selon la revendication 13, caractérisé en ce que le dispositif (6) est réalisé sous forme de digesteur de balles.

15. Dispositif selon la revendication 13, caractérisé en ce que le dispositif (6) est réalisé sous forme de silo de décomposition clos automatisé.

16. Dispositif selon une des revendications 13 - 15, caractérisé en ce que le dispositif (6) est raccordé à un dispositif de ventilation forcée (7).

17. Dispositif selon une des revendications 13 - 16, caractérisé en ce qu'un échangeur de chaleur (8) pour le chauffage des plantes aquatiques est agencé entre le dispositif hacheur (1) et le réacteur (2).

18. Dispositif selon la revendication 17, caractérisé en ce que l'échangeur de chaleur (8) est mis en communication avec le côté d'échappement de l'air du dispositif (6).

19. Dispositif selon la revendication 17 ou 18, caractérisé en ce que le côté sortie du biogaz du réacteur (2) est mis en communication avec une installation de chauffe (9) pour chauffer l'échangeur de chaleur (8).

20. Dispositif selon une des revendications 13 - 19, caractérisé en ce que le réacteur (2) est réalisé sous forme de réacteur de décomposition à deux étapes.

EP 0 227 584 B1